# EUROPEAN PATENT APPLICATION

(11) **EP 4 650 751 A1**
(43) Date of publication of application: **19.11.2025**
(21) Application number: 23916370.2
(22) Date of filing: 31.05.2023
(51) Int. Cl.: G01N 21/25, G01N 21/84, C12M 1/34, C12M 1/00, C12M 3/00, C12M 3/06

(54) **OPTICAL ANALYZER AND BIOREACTOR COMPRISING SAME**

(30) Priority: 12.01.2023 KR 20230004843
(71) Applicant: Cubick, Seoul 03911 (KR)
(72) Inventor: KIM, Dongchoul, Seoul 04346 (KR); KANG, Taewook, Seoul 07977 (KR); MIN, Junwon, Seoul 05812 (KR)
(74) Representative: Witte, Weller & Partner Patentanwälte mbB
(86) International application number: PCT/KR2023/007412
(87) International publication number: WO 2024/150886

(57) **Abstract**

An optical analyzer and a bioreactor comprising same are disclosed. The optical analyzer according to one embodiment of the present invention may comprise: a light supply unit including a light source emitting light; a probe which emits light at an analyte, and includes, at one end thereof, a detection unit for acquiring the light that penetrated the analyte; an analysis unit for analyzing the analyte on the basis of characteristics of the light acquired by the detection unit; and a bubble blocking member, which includes a fluid outlet provided to be adjacent to the detection unit and a fluid inlet provided to be spaced farther than the fluid outlet from the detection unit in the longitudinal direction from the fluid outlet and is fastened at one end of the probe.

## Description

### BACKGROUND

### 1. Field

The present invention relates to an optical analyzer and a bioreactor including the same, and more particularly, to an optical analyzer which can prevent optical signal interference and concentration measurement errors caused by bubbles when an analyte is analyzed through a spectral signal obtained from the analyte within a bioreactor, and a bioreactor including the same.

### 2. Description of the Related Art

In general, bioreactors are widely used in the bio-industry as biological reactions or processes can be performed on a laboratory scale or industrial scale.

Products that can be produced using the bioreactors may include foods and beverages, pharmaceuticals, bio-based chemicals, plastics, biofuels, and a variety of substances derived from cell cultures.

Meanwhile, when cell cultures are grown using such bioreactors, a typical method for monitoring the cell cultures is to take samples from the bioreactor at preset time intervals and analyze the samples. However, such conventional monitoring methods have problems that samples taken externally from the bioreactor may be contaminated, and rapid changes occurring in the cell cultures during periods when monitoring is not performed cannot be detected.

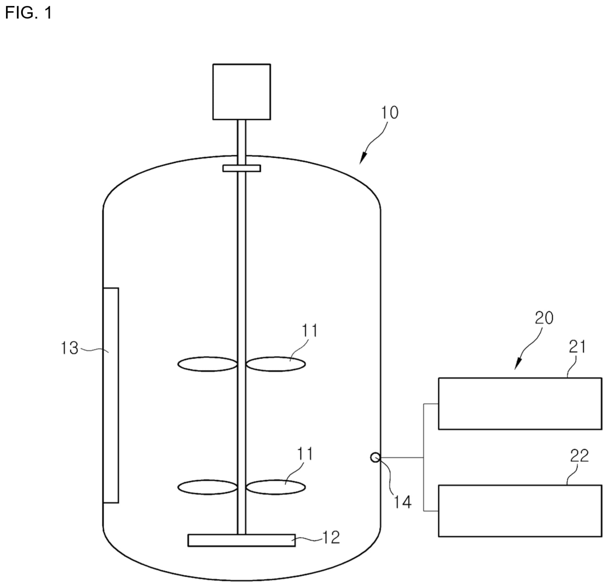

To solve these problems, a cell culture proliferation system including a reactor 10 and an optical analyzer 20 has been recently used to monitor cell cultures in real time, as shown in FIG. 1. Here, the reactor 10 includes a plurality of impellers 11 which mix stored cell suspension and nutrient materials, a sparger or aeration 12 which is a device provided at an inner lower portion thereof to disperse a gas in a liquid, a baffle 13 provided on an inner side wall thereof, a port 14 through which a sample can be taken from the reactor 10 to the outside or a probe of an analyzer can be inserted, and the like. Further, the optical analyzer 20 includes a light source 21 provided outside the reactor 10, an analyzer 22, and a probe (not shown) that may be inserted into the reactor 10 through the port 14.

However, in the cell culture proliferation system, a large amount of bubbles are generated from a fluid medium and a cell suspension, which contain liquid and gas supplied and stored inside the reactor 10, by the impeller 11 and the sparger or aeration 12.

Therefore, in this cell culture proliferation system, there is a problem that, when the probe is inserted into the reactor 10 and an optical analysis is performed by the optical analyzer 20, reliability of the analysis is lowered because interference of an optical signal occurs due to scattering of light by the bubbles, which may cause errors in concentration measurement.

### SUMMARY

An embodiment is directed to providing an optical analyzer capable of continuously monitoring a cell culture inside a reactor without taking samples to the outside of the reactor, and a bioreactor including the same.

An embodiment is directed to providing an optical analyzer capable of blocking bubbles present inside a reactor being introduced to the probe of the optical analyzer and preventing interference of an optical signal due to the bubbles, and a bioreactor including the same.

One embodiment of the present invention provides an optical analyzer including a light supply unit including a light source that emits light, a probe which irradiates an analyte with the light and of which one end is provided with a detection unit to acquire light transmitted through the analyte, an analysis unit configured to analyze the analyte based on characteristics of the light acquired by the detection unit, and a bubble blocking member fastened to the one end of the probe and including a fluid outlet provided adjacent to the detection unit and a fluid inlet provided to be spaced farther than the fluid outlet from the detection unit in a longitudinal direction from the fluid outlet.

The bubble blocking member may include a light-transmitting material.

The bubble blocking member may have a hollow cylindrical shape of which one end portion, in which the fluid inlet is provided, is closed, and the other end portion, in which the fluid outlet is provided, is open.

Each of the fluid inlet and the fluid outlet may be provided in a slit shape in a circumferential surface of the bubble blocking member.

An area of the fluid inlet may be provided to be larger than an area of the fluid outlet.

Openings of the fluid inlet and the fluid outlet may be provided in the same direction.

A distance between the fluid inlet and the fluid outlet may be determined according to a flow rate of a fluid containing the analyte.

An area of each of the fluid inlet and the fluid outlet may be determined according to a flow rate of a fluid containing the analyte.

The bubble blocking member may further include a groove or protrusion-shaped guiding structure configured to guide a fluid introduced through the fluid inlet to the fluid outlet.

Another embodiment of the present invention provides a bioreactor including any one of the optical analyzers.

An optical analyzer and a bioreactor including the same according to embodiments of the present invention can improve the reliability and continuity of analysis results by continuously monitoring cell cultures inside the reactor without taking a sample to the outside of the reactor.

This embodiment can improve accuracy of the analysis results by blocking bubbles present inside the reactor being introduced to the probe of the optical analyzer and preventing interference of an optical signal due to the bubbles.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a diagram illustrating main components of a conventional system for proliferating cell cultures.
FIG. 2 is a diagram illustrating main components of an optical analyzer according to one embodiment of the present invention.
FIGS. 3A and 3B are cross-sectional views of a bubble blocking member coupled to one end of a probe of the optical analyzer according to one embodiment of the present invention.
FIG. 4 is a diagram illustrating main components of a bioreactor including the optical analyzer according to one embodiment of the present invention.
FIG. 5 is a diagram illustrating a flow of liquid and gas introduced into and discharged from a bubble blocking member of the optical analyzer according to one embodiment of the present invention.

### DETAILED DESCRIPTION

Hereinafter, embodiments of the present invention will be described in detail with reference to the accompanying drawings. The following embodiments are presented to sufficiently convey the idea of the present invention to those skilled in the art to which the present invention pertains. The present invention is not limited to the embodiments presented herein and may be embodied in other forms. In order to clarify the present invention, the drawings may omit parts that are not related to the description and may slightly exaggerate the sizes of components to help understanding.

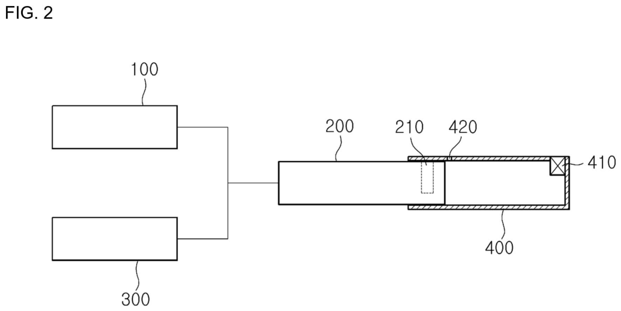

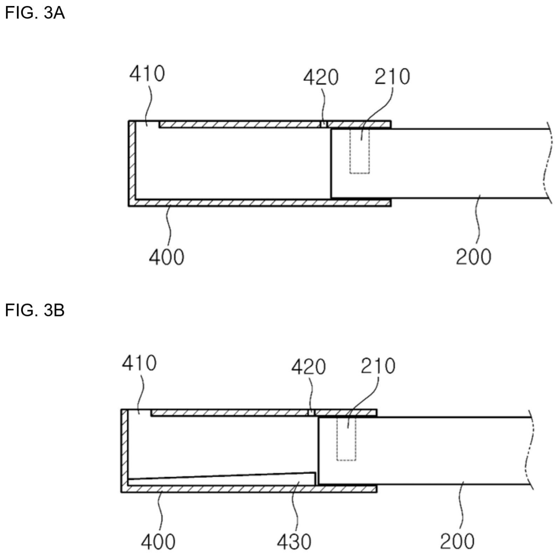

FIG. 2 is a diagram illustrating main components of an optical analyzer according to one embodiment of the present invention, and FIGS. 3A and 3B are cross-sectional views of a bubble blocking member coupled to one end of a probe of the optical analyzer according to one embodiment of the present invention.

Referring to FIGS. 2 and 3, the optical analyzer according to the embodiment of the present invention includes a light supply unit 100, a probe 200, an analysis unit 300, and a bubble blocking member 400.

The light supply unit 100 includes a light source that emits light. Here, the light may be various types of light, such as light in an infrared region (IR) including light in a near infrared region (NIR), light in an X-ray region (XRD), and light in a radio wave region (NMR). Additionally, the light supply unit 100 may be a multi-wavelength laser that emits light having multiple different wavelengths in the near-infrared region.

The entirety of the probe 200 has a rod shape and includes a detection unit 210 provided on one end thereof and into which a fluid containing an analyte is introduced. More specifically, the probe 200 may be provided to irradiate an analyte with light through the detection unit 210 and obtain light transmitted through the analyte. Additionally, the probe 200 may have a waterproof function to prevent damage due to introduction of liquid.

The analysis unit 300 is a means for analyzing the analyte based on characteristics of the light transmitted through the analyte and obtained by the detection unit 210 as described above, and may be provided to analyze a concentration of the analyte, or the like, through various analysis methods already known.

The bubble blocking member 400 is fastened to one end of the probe 200 and includes a fluid outlet 420 provided adjacent to the detection unit 210 and a fluid inlet 410 provided to be spaced farther than the fluid outlet 420 from the detection unit 210 in a longitudinal direction from the fluid outlet 420. In this case, the bubble blocking member 400 may be provided integrally with or separately from the probe 200 and may be provided as a light-transmitting material.

More specifically, the bubble blocking member 400 may be provided in a hollow cylindrical shape of which one end portion, in which the fluid inlet 410 is provided, is closed, and the other end, in which the fluid outlet 420 is provided, is open. In this case, each of the fluid inlet 410 and the fluid outlet 420 may be provided in a slit shape on a circumferential surface of the bubble blocking member 400.

As illustrated in FIG. 3B, the bubble blocking member 400 may further include a groove or protrusion-shaped guiding structure 430 that guides a fluid introduced through the fluid inlet 410 to the fluid outlet 420.

Here, the term "fluid" refers to a fluid that includes an analyte to be analyzed by an optical analyzer according to one embodiment of the present invention and may include various fluid media in a gaseous or liquid state.

The guiding structure 430 may be a protrusion or groove provided in the longitudinal direction inside the bubble blocking member 400 and may also be a spiral-shaped protrusion or groove. However, the guiding structure 430 is not designed to have a complex shape that may cause the fluid introduced into the bubble blocking member 400 to stagnate or generate bubbles.

Meanwhile, an area of the fluid inlet 410 may be provided to be larger than an area of the fluid outlet 420. In addition, openings of the fluid inlet 410 and the fluid outlet 420 may be provided in the same direction.

A distance between the fluid inlet 410 and the fluid outlet 420 may be determined according to a flow rate of the fluid containing the analyte. For example, when it is used in a space in which the flow rate of the fluid is formed rapidly, the distance between the fluid inlet 410 and the fluid outlet 420 may be provided to be larger than when it is used in a space in which the flow rate of the fluid is formed relatively slowly.

Additionally, the area of each of the fluid inlet 410 and the fluid outlet 420 may be determined according to the flow rate of the fluid containing the analyte. For example, when it is used in the space in which the flow rate of the fluid is formed rapidly, the area of each of the fluid inlet 410 and the fluid outlet 420 may be made larger than when it is used in the space in which the flow rate of the fluid is formed relatively slowly.

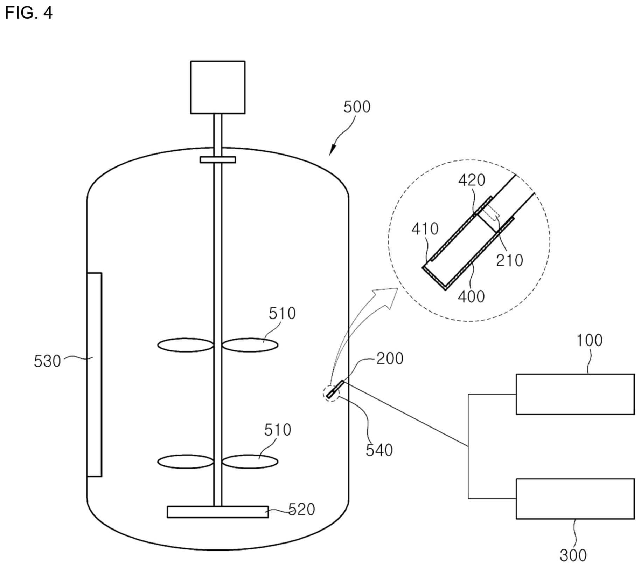

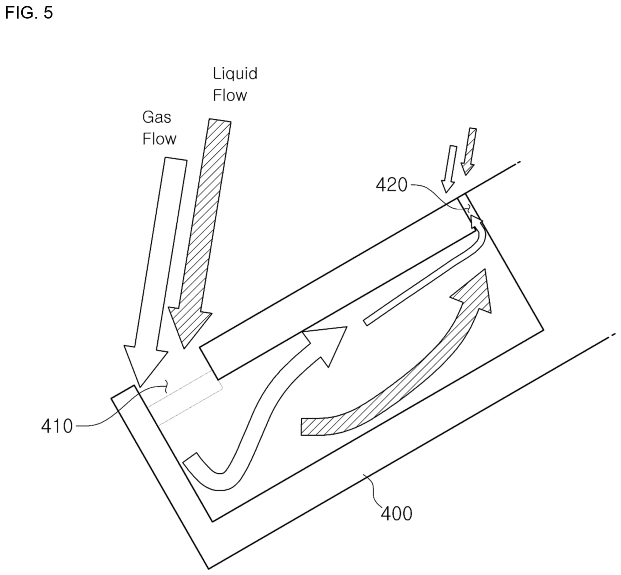

Meanwhile, FIG. 4 is a diagram illustrating main components of a bioreactor including the optical analyzer according to the embodiment of the present invention, and FIG. 5 is a diagram illustrating a flow of liquid and gas introduced into and discharged from a bubble blocking member when the optical analyzer according to the embodiment of the present invention is disposed in the bioreactor.

Referring to FIGS. 4 and 5, the bioreactor 500 including the optical analyzer according to the embodiment of the present invention has a space provided therein for accommodating a cell culture and a fluid medium.

Similar to the previously known bioreactor, the bioreactor 500 may include a plurality of impellers 510 that mix a stored cell suspension and nutrient materials, a sparger or aeration 520 provided at an inner lower portion to disperse gas in a liquid, a baffle 530 provided on an inner side wall, and a port 540 provided to take a sample to the outside or to allow a probe 200 or the like to be inserted or discharged.

In addition, although not illustrated, the bioreactor 500 may include a medium supply unit that supplies various fluid media for growth of the cell culture, a waste discharge unit that discharges waste or the like generated during the growth of the cell cultures, and the like.

Furthermore, it goes without saying that various known structures in which liquid and gas are stored and cell cultures are grown may be applied to the bioreactor 500.

Accordingly, the optical analyzer and the bioreactor including the same according to the embodiment of the present invention can improve reliability and continuity of analysis results by continuously monitoring the cell cultures inside the reactor without taking a sample to the outside of the reactor.

In addition, in the probe 200 to which the bubble blocking member 400 provided into the bioreactor 500 is fastened, one end of the bubble blocking member 400 is lifted upward by buoyancy caused by gas introduced into the inside of the bubble blocking member 400.

In addition, although FIG. 4 illustrates that the light supply unit 100 and the analysis unit 300 are provided separately outside the bioreactor 500, it goes without saying that all components of the optical analyzer according to the embodiment of the present invention may be provided integrally with the bioreactor 500.

For example, the bioreactor 500 may have a cylindrical shape having a diameter of 7700 mm and a height of 9400 mm, and a maximum volume of an internal space thereof may be 400 kL. Additionally, bubbles of 200 Nm3/min may be supplied to the internal space of the bioreactor 500 from a ring-shaped aeration 520 having a pipe diameter of 100 mm and a total diameter of 1750 mm. In addition, there are eight impellers in total, every two impellers 510 having the same height are installed at preset intervals in a height direction, and a rotation speed may be set to 60 rev/min.

In this case, at the port 540 of the bioreactor 500, that is, in a region in which detection of the analyte is performed by the probe 200 of the optical analyzer according to the embodiment of the present invention, a flow rate of a liquid was measured as 3.07 m/s, a flow rate of a gas was measured as 2.72 m/s, and a gas volume fraction was measured as 0.17.

Accordingly, when an optical analysis is performed using the probe 200 in a state in which the bubble blocking member 400 is not installed, it could be expected that accuracy of the analysis would be reduced due to interference of light caused by bubbles.

In the bubble blocking member 400 of the optical analyzer according to the embodiment of the present invention, which is provided on the port 540, liquid and gas stored in the bioreactor 500 is continuously introduced through the fluid inlet 410 according to a flow formed by the aeration 520 and the impeller 510 as described above, and as shown in FIG. 5, the end portion at the fluid inlet 410 is maintained in an inclined state due to buoyancy. In addition, it was confirmed that, when the bubble blocking member 400 was not installed, the gas volume fraction at the detection unit 210 was measured as 0.12, but when the bubble blocking member 400 was installed at the probe 200, the gas volume fraction at the detection unit 210 was measured as 0.034.

That is, according to the optical analyzer according to the embodiment of the present invention, it was confirmed that bubbles were reduced by 70% or more in the detection unit 210 by the bubble blocking member 400 being provided. In other words, it can be seen that an amount of bubbles contained in the fluid introduced into the detection unit 210 is significantly smaller than an amount of bubbles contained in the fluid introduced through the fluid inlet 410.

Meanwhile, under the same conditions as described above, when the probe 200 had a cylindrical shape having a length of 128 mm and a diameter of 18 mm, and the distance between the fluid inlet 410 and the fluid outlet 420 was 50 mm, a small amount of bubbles were measured, but when the distance was 70 mm or more, no bubbles were measured. As a result, as described above, it was confirmed that the distance between the fluid inlet 410 and the fluid outlet 420 was formed at a sufficient distance and could be determined according to the flow rate of the fluid containing the analyte.

Accordingly, the present embodiment can improve the accuracy of the analysis results by blocking bubbles present inside the bioreactor 500 being introduced to the probe 200 of the optical analyzer and preventing interference of an optical signal due to bubbles.

Although specific embodiments of the optical analyzer of the present invention and the bioreactor including the same have been described so far, it is obvious that various modifications are possible within the scope of the present invention.

Therefore, the scope of the present invention should not be limited to the described embodiments, but should be defined not only by the claims set forth below but also by equivalents of the claims.

That is, it should be understood that the above-described embodiments are exemplary in all respects and not restrictive, the scope of the present invention is indicated by the patent registration claims to be described below rather than by the detailed description, and all changes or modifications derived from the meaning and scope of the patent registration claims and their equivalent concepts should be interpreted as being included in the scope of the present invention.

## Claims

1. An optical analyzer comprising:
a light supply unit including a light source that emits light;
a probe which irradiates an analyte with the light and of which one end is provided with a detection unit to acquire light transmitted through the analyte;
an analysis unit configured to analyze the analyte based on characteristics of the light acquired by the detection unit; and
a bubble blocking member fastened to the one end of the probe and including a fluid outlet provided adjacent to the detection unit and a fluid inlet provided to be spaced farther than the fluid outlet from the detection unit in a longitudinal direction from the fluid outlet.

2. The optical analyzer of claim 1, wherein the bubble blocking member includes a light-transmitting material.

3. The optical analyzer of claim 1, wherein the bubble blocking member has a hollow cylindrical shape of which one end portion, in which the fluid inlet is provided, is closed, and the other end portion, in which the fluid outlet is provided, is open.

4. The optical analyzer of claim 3, wherein each of the fluid inlet and the fluid outlet is provided in a slit shape in a circumferential surface of the bubble blocking member.

5. The optical analyzer of claim 1, wherein an area of the fluid inlet is provided to be larger than an area of the fluid outlet.

6. The optical analyzer of claim 1, wherein openings of the fluid inlet and the fluid outlet are provided in the same direction.

7. The optical analyzer of claim 1, wherein a distance between the fluid inlet and the fluid outlet is determined according to a flow rate of a fluid containing the analyte.

8. The optical analyzer of claim 1, wherein an area of each of the fluid inlet and the fluid outlet is determined according to a flow rate of a fluid containing the analyte.

9. The optical analyzer of claim 1, wherein the bubble blocking member further includes a groove or protrusion-shaped guiding structure configured to guide a fluid introduced through the fluid inlet to the fluid outlet.

10. A bioreactor including the optical analyzer of any one of claims 1 to 9.
